# FASCICULE DE BREVET EUROPEEN

(11) **EP 0 699 427 B1**
(45) Date de publication et mention de la délivrance du brevet: **02.06.1999**
(21) Numéro de dépôt: 95401624.2
(22) Date de dépôt: 06.07.1995
(51) Int. Cl.: A61F 13/15, A61F 15/00

(54) **Protection périodique enroulée**
Eingerollte Monatsbinde
Individually rolled sanitary towel

(30) Priorité: 08.07.1994 FR 9408482
(43) Date de publication de la demande: 06.03.1996
(73) Titulaire: FORT JAMES FRANCE, 68320 Kunheim (FR)
(72) Inventeur: Lefebvre du Grosriez, Carol, F-78600 Maisons Lafitte (FR)
(74) Mandataire: David, Daniel

(56) Documents cités:
- FR-A- 2 638 426
- US-A- 4 579 556

## Description

La présente invention concerne un article d'hygiène féminine, notamment de type serviette périodique, protège-slip ou similaire, et destiné à être porté avec un sous-vêtement de maintien.

De tels articles sont bien connus dans le métier et de nombreux documents de brevet en décrivent. On peut ainsi citer, parmi tant d'autres, FR-A-2 632 855, FR-A-2 636 522 et FR-A-2 678 163 au nom du présent déposant. D'une façon générale, un tel article comprend un matelas absorbant de préférence de faible épaisseur et de forme allongée, disposé entre une couche de surface perméable aux fluides corporels et une couche imperméable à ces fluides. La couche imperméable comporte, sur sa face extérieure, un moyen de fixation tel qu'un adhésif destiné à faire adhérer momentanément cette face extérieure à une partie d'un sous-vêtement. Un moyen de protection du moyen de fixation par exemple une bande pelable dans le cas d'un adhésif, protège le moyen de fixation contre toute adhésion avant utilisation. L'article est emballé avant utilisation dans un étui de protection pouvant servir également d'emballage pour envelopper l'article après usage.

Ces articles sont devenus des produits de très grande consommation dont la fabrication doit être la plus automatisée possible et la plus économique possible, sans affecter leurs propriétés et, au contraire, en y apportant de nouveaux avantages. Ainsi, on a cherché à simplifier et même à éliminer l'étui de protection lorsqu'il s'agissait d'un composant supplémentaire, par exemple une pochette individuelle en film plastique tel qu'un film de polyéthylène. Toutefois, subsiste alors le problème de l'emballage de l'article avant et après utilisation, l'emballage restant nécessaire et devant être de manipulation aisée.

Par ailleurs, pour le transport et le stockage de ces produits, un encombrement réduit est recherché.

Enfin, l'article d'hygiène emballé avant utilisation dans une pochette reste de dimensions non négligeables. L'utilisatrice est toujours soucieuse de plus de discrétion en pouvant dissimuler un article enveloppé dans un emballage de petites dimensions.

Le document EP-A-0 269 879 propose une solution sans étui où l'article d'hygiène est enroulé sur lui-même avant utilisation au moyen d'un cordon disposé dans un canal ménagé sur chaque bord longitudinal du voile imperméable.

Le cylindre formé, dont les bords sont fermés, permet de protéger de manière hermétique la surface de l'article avant utilisation. Toutefois, les cordons qui, par ailleurs, ne sont pas agréables pour l'utilisatrice, constituent un surcoût tant en matière qu'en temps de fabrication, surcoût qu'il y a eu lieu d'éviter, même si l'on peut ainsi éliminer l'emballage de l'article.

Le document EP-A-0 357 000 décrit une solution selon laquelle l'article est enroulé ou plié le long d'au moins un axe transversal. L'article comprend une feuille enveloppante allongée adhérant de façon amovible à au moins une partie de la face du matelas absorbant comportant des moyens adhésifs, la feuille enveloppante ayant sensiblement la même longueur que le matelas et s'étendant au-delà d'une extrémité de ce matelas pour former un volet ou rabat. L'article est ainsi enveloppé dans la bande pelable. Or, cette bande pelable, généralement siliconée pour favoriser le pelage, est un composant coûteux par rapport à l'article, composant dont il y a eu lieu de limiter l'utilisation.

La demande de brevet internationale WO-A-93/09743 propose également une pochette en matériau formant directement bande siliconée et comprenant un volet ou rabat muni d'une languette. L'article plié en trois à plat est emballé au moyen de ce matériau avant utilisation. La longueur de l'enveloppe formant bande siliconée reste toujours supérieure à celle de l'article. Après utilisation, l'article peut être enroulé dans le matériau formant enveloppe.

La présente invention a pour but de pallier les inconvénients de l'art antérieur, en réduisant les composants et donc les coûts de fabrication d'un tel article externe d'hygiène féminine et en fournissant un article emballé de plus petite dimension.

L'invention a donc pour objet, un article externe d'hygiène féminine tel qu'une serviette périodique, comprenant un matelas absorbant de préférence de faible épaisseur et de forme allongée, disposé entre une couche perméable aux fluides corporels et une couche imperméable à ces fluides, la couche imperméable portant, sur sa face extérieure, un moyen de fixation tel qu'un moyen adhésif destiné à faire adhérer momentanément cette face extérieure à une partie d'un sous-vêtement et au moins partiellement protégé avant utilisation contre toute adhésion par un moyen de protection tel qu'une bande pelable, cet article étant enfermé avant utilisation dans un étui formé par le moyen de protection et étant enroulé sur lui-même sur plusieurs tours de manière à former substantiellement un cylindre.

Selon l'invention, le moyen de protection présente une longueur sensiblement égale à la circonférence du cylindre ainsi formé.

De préférence, le moyen de protection présente une largeur sensiblement supérieure à celle du matelas et ses bords sont scellés entre eux en regard de chaque base du cylindre.

De préférence également, une tirette est enroulée avec l'article, tirette constituée par un fil ou par un ruban, et présentant une résistance à la traction suffisante pour dépasser la force d'adhésion générée par le moyen de fixation au contact de la couche perméable de surface.

Selon une caractéristique supplémentaire, au moins une languette de préhension solidaire de l'un des composants de l'article fait saillie à l'extérieur du cylindre.

Le moyen de fixation peut être un adhésif favorablement constitué par une couche d'une colle adhésive par pression.

L'invention sera mieux comprise, et d'autres buts, avantages et caractéristiques de celle-ci apparaîtront plus clairement à la lecture de la description qui suit de modes préférés de réalisation donnés à titre non limitatif et à laquelle une planche de dessins est annexée sur laquelle :
- la figure 1 représente schématiquement en perspective et partiellement ouvert un article conforme à la présente invention selon un premier mode préféré de réalisation ;
- la figure 2 représente schématiquement en perspective et partiellement ouvert un article conforme à la présente invention selon un autre mode préféré de réalisation ; et
- la figure 3 représente schématiquement en perspective un article dans son étui selon la présente invention.

En référence maintenant aux dessins, on décrira certains modes préférés de réalisation de l'invention. L'article externe d'hygiène féminine, dans la suite de la description, sera une protection périodique, bien que d'autres applications de l'invention soient envisagées. Cet article est essentiellement constitué d'un matelas de préférence ultra-mince 2, par exemple en mousse de cellulose ou en tout autre matériau absorbant, de forme allongée, disposé entre une couche de surface (non référencée sur les figures) perméable aux fluides corporels portant, par exemple, des motifs en relief, notamment pour améliorer la perméabilité par capillarité, et une couche imperméable aux fluides 4.

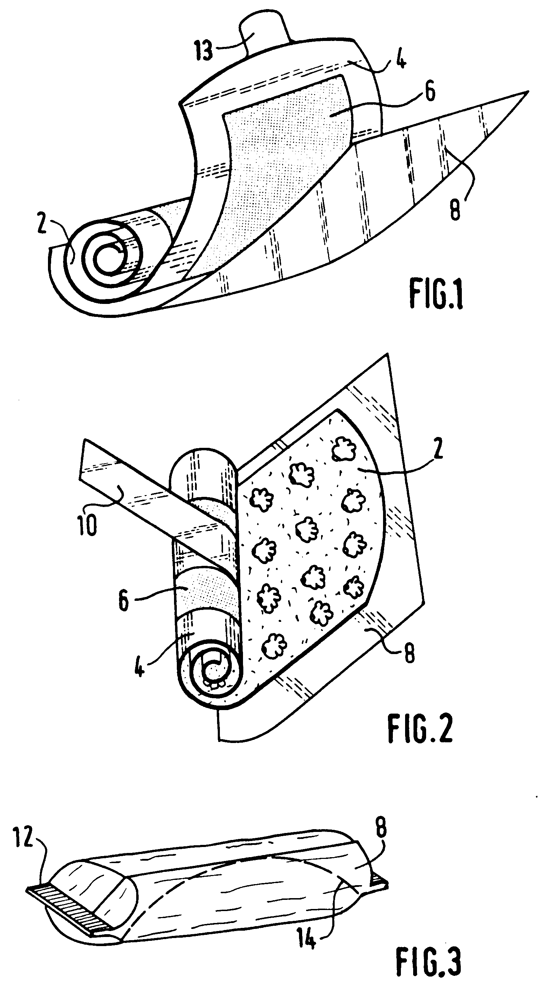

Dans un mode de réalisation préféré, le matériau absorbant est formé de fibres cellulosiques contenant une matière superabsorbante sous forme de poudre. Par exemple, une bande de fibres cellulosiques, papetières, formée par voie sèche et liée par un latex, dont les deux bords latéraux sont rabattus sur une partie centrale sur laquelle est déposée au préalable le matériau surperabsorbant, peut former ce type de matelas. Un tel matelas est représenté en figure 3. Le matériau superabsorbant peut être sous forme de poudre, de feuille et également de poudre liée à des fibres synthétiques. On peut également envisager un matelas constitué d'un mélange de fibres cellulosiques et de fibres synthétiques, placé entre deux couches d'ouate de cellulose, l'ensemble étant thermolié.

D'autres matériaux tels qu'une mousse synthétique, par exemple de polyuréthane, ou naturelle, par exemple de sphaigne traitée, présentant de grandes capacités d'absorption, peuvent être utilisés. Le traitement de la sphaigne mentionnée ci-dessus est décrit dans une demande de brevet européen n° 0 546 585.

La couche ou voile de surface peut être réalisée dans un matériau nontissé ou dans un film de plastique perforé, par exemple en polyéthylène, présentant des micro-perforations.

La couche imperméable située sous le matelas absorbant est un matériau plastique imperméable, tel que le polyéthylène ou tout matériau équivalent imperméable. Elle peut également être constituée d'un matériau étant à la fois perméable à l'air et à la vapeur d'eau et imperméable aux liquides. Un tel matériau est par exemple un non-tissé de type "meltblown" composé de microfibres, par exemple de polyéthylène. La couche imperméable pourrait encore être constituée d'une mousse de polyuréthane ou tout matériau équivalent.

Un moyen de fixation 6, ici un moyen adhésif, est disposé, par exemple en pavé, sur la face extérieure de manière à ce que l'article adhère à une partie d'un sous-vêtement. Dans l'art antérieur, avant utilisation de l'article, ce moyen adhésif est totalement recouvert d'un moyen de protection tel qu'une bande pelable, par exemple siliconée, permettant d'éviter toute adhésion intempestive de l'article sur une surface non prévue à cet effet. Cette bande est ôtée avant la mise en place de l'article sur le sous-vêtement.

Pour mettre en oeuvre l'invention, l'article est enroulé sur lui-même sur plusieurs tours de manière à former substantiellement un cylindre. L'enroulement peut se faire selon tout axe de l'article, mais un enroulement dans un axe transversal est préféré pour minimiser les dimensions du cylindre obtenu. La bande pelable 8 est disposée à l'extérieur du cylindre et non sur toute la longueur de la couche imperméable. Elle n'est donc enroulée que lorsque l'article est lui-même déjà en partie enroulé, et ne présente alors qu'une longueur sensiblement égale à la circonférence du cylindre. Mais, de ce fait, le moyen adhésif disposé sur la face extérieure de l'article est en contact de la couche de surface ou voile perméable 2 de l'article.

On choisit donc judicieusement le moyen adhésif pour qu'il n'adhère que très peu au voile perméable, tout en adhérant parfaitement au sous-vêtement ultérieurement. De tels moyens adhésifs sont, par exemple, décrits dans EP-A-0 393 953, EP-A-0 368 102 et EP-A-0 573 044. Une couche de colle adhésive par pression reste préférée.

Selon un mode préféré de réalisation de l'invention, la bande pelable 8 présente une largeur sensiblement supérieure à celle du matelas 2, de telle manière que les bords 12 de cette bande puissent être scellés entre eux en regard de chaque base du cylindre, par exemple par thermo-soudure, comme représenté en figure 3. Une pré-découpe 14 peut être favorablement prévue pour faciliter l'ouverture de l'ensemble. Ainsi scellé dans son étui, l'article peut présenter toutes les qualités requises de propreté et d'hygiène pour une bonne utilisation. En outre, l'utilisatrice n'a pas à déchirer les extrémités de l'étui, ce qui simplifie beaucoup l'ouverture de l'article.

Lorsque l'étui est ouvert par l'utilisatrice, il est préférable qu'elle puisse dérouler l'article sans aucune manipulation difficile. Dans ce but, comme représenté en figure 2, une tirette 10 est favorablement enroulée avec l'article pour former le cylindre. Cette tirette a été représentée sous forme d'une bande, mais peut également être constituée par un simple fil. L'utilisatrice, après ouverture de l'étui, peut donc, en tenant entre deux doigts d'une main l'article enroulé et entre deux doigts de l'autre main la tirette 10, ouvrir complètement l'article en vue de le disposer sur le sous-vêtement.

Cette tirette 10 doit donc présenter une résistance à la traction suffisante pour dépasser la force d'adhésion résiduelle générée par le moyen adhésif 6 au contact du voile perméable, de manière à ne pas se rompre lors de son utilisation.

Suivant une caractéristique supplémentaire de l'invention, comme représenté en Figure 1, une languette de préhension 13 solidaire de l'un des composants de l'article peut être prévue. Cette languette de préhension 13 fera alors saillie à l'extérieur du cylindre. On peut favorablement prévoir une première languette 13 solidaire de la couche imperméable 4 et une deuxième languette (non représentée) solidaire de la bande pelable 8. Ainsi, en tirant simultanément sur les deux languettes en regard, l'utilisatrice peut facilement séparer les deux composants de l'article.

Bien évidemment, ces languettes peuvent être associées à la tirette précitée pour assurer une ouverture optimale de l'article conformément aux désirs de l'utilisatrice.

On constate donc que l'invention apporte un article d'hygiène d'encombrement minimal permettant sa dissimulation facile dans la main. En outre, cet article est de manipulation très aisée. Après usage, l'article peut être à nouveau enroulé sur luimême et être remis dans l'étui faisant alors office d'emballage avant d'être jeté.

De toute évidence, l'article peut être pourvu de volets latéraux repliés normalement sur la couche perméable. L'article peut également comporter des moyens supplémentaires tels que des barrières latérales, des bordures latérales élastifiées, des bordures douces dans la zone d'entrejambes, etc... pour améliorer les performances du produit ou son confort.

En outre, l'enroulement de l'article favorise avantageusement une forme légèrement incurvée de l'article après déroulement.

Comme l'homme du métier peut le constater, l'invention apporte une économie de matière très importante par élimination de l'étui conventionnel et réduction de la taille de la bande pelable siliconée. Il en résulte à l'évidence un coût de fabrication réduit tout en maintenant les qualités et avantages des articles ainsi réalises.

Selon une variante de l'invention encore plus économique, le moyen de protection est collé de façon permanente à la couche imperméable. Dans ce cas, l'ouverture de l'article se fait comme représenté en Figure 2. Le moyen de protection n'est alors pas constitué par un matériau siliconé coûteux, mais sera en un simple matériau non irritant et non bruyant. La surface du pavé portant le moyen adhésif 6 reste suffisante pour une borne fixation de l'article sur le sous-vêtement.

Bien que l'on ait représenté et décrit ce que l'on considère actuellement être les modes de réalisation préférés de la présente invention, il est évident que l'homme de l'art pourra y apporter différents changements et modifications sans sortir du cadre de la présente invention tel que défini par les revendications jointes.

## Revendications

1. Article externe d'hygiène féminine, tel qu'une serviette périodique, comprenant un matelas absorbant (2) de préférence de faible épaisseur et de forme allongée, disposé entre une couche de surface perméable aux fluides corporels et une couche imperméable (4) auxdits fluides, ladite couche imperméable (4) comportant, sur sa face extérieure, un moyen de fixation (6) destiné à faire adhérer momentanément ladite face extérieure à une partie d'un sous-vêtement et au moins partiellement protégé contre toute adhésion avant utilisation par un moyen de protection (8) tel qu'une bande pelable, ledit article étant emballé avant utilisation dans un étui formé par ledit moyen de protection et étant enroulé sur lui-même sur plusieurs tours de manière à former substantiellement un cylindre, caractérisé en ce que ledit moyen de protection (8) présente une longueur sensiblement égale à la circonférence dudit cylindre.

2. Article selon la revendication 1, caractérisé en ce que le moyen de protection (8) présente une largeur sensiblement supérieure à celle dudit matelas (2).

3. Article selon la revendication 2, caractérisé en ce que les bords (12) dudit moyen de protection (8) sont scellés entre eux en regard de chaque base dudit cylindre.

4. Article selon l'une quelconque des revendications précédentes, caractérisé en ce qu'une tirette (10) est enroulée avec ledit article.

5. Article selon la revendication 4, caractérisé en ce que ladite tirette (10) est constituée par un fil.

6. Article selon la revendication 4, caractérisé en ce que ladite tirette (10) est constituée par un ruban.

7. Article selon l'une quelconque des revendications 4 à 6, caractérisé en ce que ladite tirette (10) présente une résistance à la traction suffisante pour dépasser la force d'adhésion générée par ledit moyen adhésif (6) au contact de ladite couche perméable.

8. Article selon l'une quelconque des revendications précédentes, caractérisé en ce qu'au moins une languette de préhension (13) solidaire de l'un des composants dudit article fait saillie à l'extérieur dudit cylindre.

9. Article externe d'hygiène féminine, tel qu'une serviette périodique, comprenant un matelas absorbant (2) de préférence de faible épaisseur et de forme allongée, disposé entre une couche de surface perméable aux fluides corporels et une couche imperméable (4) auxdits fluides, ladite couche imperméable (4) comportant, sur sa face extérieure, un moyen de fixation (6) destiné à faire adhérer momentanément ladite face extérieure à une partie d'un sous-vêtement et un moyen de protection (8), ledit article étant emballé avant utilisation dans un étui formé par ledit moyen de protection et étant enroulé sur lui-même sur plusieurs tours de manière à former substantiellement un cylindre, caractérisé en ce que ledit moyen de protection (8) est collé de façon permanente à ladite couche imperméable (4) et présente une longueur sensiblement égale à la circonférence dudit cylindre.

10. Article selon l'une quelconque des revendications précédentes, caractérisé en ce que ledit moyen de fixation (6) est un adhésif constitué par une couche d'une colle adhésive par pression.

11. Article selon l'une quelconque des revendications précédentes, caractérisé en ce que ledit article est pourvu de volets latéraux.

## Claims

1. External article of feminine hygiene, such as a sanitary towel, comprising an absorbent pad (2) which is preferably thin and elongated in shape, disposed between a surface layer permeable to body fluids and a layer (4) impermeable to the said fluids, the said impermeable layer (4) including, on its outer surface, a means of attachment (6) designed to cause the said outer surface to adhere temporarily to a part of an undergarment and at least partially protected before use against any adhesion by a means of protection (8) such as a peelable strip, the said article being packaged before use in a holder formed by the said means of protection and being rolled up on itself in several turns so as to form substantially a cylinder, characterized in that the said means of protection (8) has a length essentially equal to the circumference of the said cylinder.

2. Article according to claim 1, characterized in that the means of protection (8) has a width essentially greater than that of the said pad (2).

3. Article according to claim 2, characterized in that the edges (12) of the said means of protection (8) are sealed together facing each base of the said cylinder.

4. Article according to any one of the preceding claims, characterized in that a pull (10) is rolled up with the said article.

5. Article according to claim 4, characterized in that the said pull (10) consists of a thread.

6. Article according to claim 4, characterized in that the said pull (10) consists of a ribbon.

7. Article according to any one of claims 4 to 6, characterized in that the said pull (10) has a tensile strength sufficient to exceed the adhesive force generated by the said adhesive means (6) in contact with the said permeable layer.

8. Article according to any one of the preceding claims, characterized in that at least one gripping tongue (13) secured to one of the components of the said article projects outside the said cylinder.

9. External article of feminine hygiene, such as a sanitary towel, comprising an absorbent pad (2) which is preferably thin and elongated in shape, disposed between a surface layer permeable to body fluids and a layer (4) impermeable to the said fluids, the said impermeable layer (4) including, on its outer surface, a means of attachment (6) designed to cause the said outer surface to adhere temporarily to a part of an undergarment and a means of protection (8), the said article being packaged before use in a holder formed by the said means of protection and being rolled up on itself in several turns so as to form substantially a cylinder, characterized in that the said means of protection (8) is permanently glued to the said impermeable layer (4) and has a length essentially equal to the circumference of the said cylinder.

10. Article according to any one of the preceding claims, characterized in that the said means of attachment (6) is an adhesive consisting of a layer of pressure sensitive adhesive.

11. Article according to any one of the preceding claims, characterized in that the said article is provided with side flaps.

## Patentansprüche

1. Externer weiblicher Hygieneartikel wie z.B. eine Monatsbinde mit einer absorbierenden Lage (2) vorzugsweise geringer Dicke und länglicher Form, die zwischen einer bezüglich Körperflüssigkeiten durchlässigen Oberflächenschicht und einer bezüglich dieser Flüssigkeiten undurchlässigen Schicht (4) angeordnet ist, wobei die undurchlässige Schicht (4) an ihrer Außenseite ein Befestigungsmittel (6) aufweist, das dazu dient, mit seiner Außenseite zeitweise an einem Abschnitt der Unterwäsche zu haften, und das zumindest partiell gegen eine Haftwirkung vor Gebrauch durch ein Schutzmittel (8) wie z.B. ein abziehbares Band geschützt ist, wobei der Artikel vor Gebrauch in einem von dem Schutzmittel gebildeten Behältnis eingehüllt und mehrere Male um sich selbst eingerollt ist, um praktisch einen Zylinder zu bilden, dadurch gekennzeichnet, daß das Schutzmittel (8) eine Länge hat, die im wesentlichen gleich dem Umfang des Zylinders ist.

2. Artikel nach Anspruch 1, dadurch gekennzeichnet, daß das Schutzmittel (8) eine Breite hat, die wesentlich größer als die der Lage (2) ist.

3. Artikel nach Anspruch 2, dadurch gekennzeichnet, daß die Ränder (12) des Schutzmittels (8) im Bereich jeder Grundfläche des Zylinders unter sich versiegelt sind.

4. Artikel nach einem der vorhergehenden Ansprüche, dadurch gekennzeichnet, daß ein Zugmittel (10) mit dem Artikel eingerollt ist.

5. Artikel nach Anspruch 4, dadurch gekennzeichnet, daß das Zugmittel (10) von einem Faden gebildet wird.

6. Artikel nach Anspruch 4, dadurch gekennzeichnet, daß das Zugmittel (10) von einem Band gebildet wird.

7. Artikel nach einem der Ansprüche 4 bis 6, dadurch gekennzeichnet, daß das Zugmittel (10) einen Zugwiderstand hat, der ausreicht, um die Adhäsionskraft, die von dem Klebmittel (6) bei Berührung mit der durchlässigen Schicht erzeugt wird, zu überwinden.

8. Artikel nach einem der vorhergehenden Ansprüche, dadurch gekennzeichnet, daß mindestens eine Grifflasche (13), die mit einem der Teile des Artikels fest verbunden ist, aus dem Zylinder nach außen vorsteht.

9. Externer weiblicher Hygieneartikel wie z.B. eine Monatsbinde mit einer absorbierenden Lage (2) vorzugsweise geringer Dicke und länglicher Form, die zwischen einer bezüglich Körperflüssigkeiten durchlässigen Oberflächenschicht und einer bezüglich dieser Flüssigkeiten undurchlässigen Schicht (4) angeordnet ist, wobei die undurchlässige Schicht (4) an ihrer Außenseite ein Befestigungsmittel (6), das dazu dient, mit seiner Außenseite zeitweise an einem Abschnitt der Unterwäsche zu haften, und ein Schutzmittel (8) aufweist, wobei der Artikel vor Gebrauch in einem von dem Schutzmittel gebildeten Behältnis eingehüllt und mehrere Male um sich selbst eingerollt ist, um praktisch einen Zylinder zu bilden, dadurch gekennzeichnet, daß das Schutzmittel (8) dauerhaft an der undurchlässigen Schicht (4) angeklebt ist und eine Länge hat, die im wesentlichen gleich dem Umfang des Zylinders ist.

10. Artikel nach einem der vorhergehenden Ansprüche, dadurch gekennzeichnet, daß das Befestigungsmittel (6) ein Kleber ist, der von einer Schicht aus einem druckempfindlichen Klebstoff besteht.

11. Artikel nach einem der vorhergehenden Ansprüche, dadurch gekennzeichnet, daß der Artikel mit seitlichen Flügeln versehen ist.
